Europäisches Patentamt

European Patent Office

Office européen des brevets

(19)

(11) Veröffentlichungsnummer: **0 065 770**
**B2**

(12) NEUE EUROPÄISCHE PATENTSCHRIFT

(45) Veröffentlichungstag der neuen Patentschrift:
12.04.89

(21) Anmeldenummer: 82104461.7

(22) Anmeldetag: 21.05.82

(51) Int. Cl.⁴: C 07 C 76/00, C 07 C 79/35

ERRATUM

(SEITE, SPALTE, ZEILE)
(PAGE, COLUMN, LINE)
(PAGE, COLONNE, LIGNE)

| DIE TEXTSTELLE :<br>TEXT PUBLISHED :<br>LE PASSAGE SUIVANT : | | | | LAUTET BERICHTIGT :<br>SHOULD READ :<br>DEVRAIT ETRE LU : |
|---|---|---|---|---|
| the reaction is carried out without any other sol- | 3 | 3 | 21 | the reaction is carried out without any other solvent at a temperature of from 60 to 70°C. |

Tag der Entscheidung
über die Berichtigung )
Date of decision on ) 17.4.90
rectification: )
Date de décision portant )
sur modification: )

Ausgabe- und Veröffentlichungstag: )
Issue and publication ) 18.7.90
date: )
Date d'edition et de )
publication: )

Patbl.Nr)
EPB no:)90/29.

Bull. no:)

## Europäisches Patentamt
## European Patent Office
## Office européen des brevets

(19)

(11) Veröffentlichungsnummer: **0 065 770**
**B2**

(12) **NEUE EUROPÄISCHE PATENTSCHRIFT**

(45) Veröffentlichungstag der neuen Patentschrift:
**12.04.89**

(51) Int. Cl.⁴: **C 07 C 76/00, C 07 C 79/35**

(21) Anmeldenummer: **82104461.7**

(22) Anmeldetag: **21.05.82**

(54) Verfahren zur Herstellung von p-Nitrophenetol.

(30) Priorität: 26.05.81 DE 3120912

(43) Veröffentlichungstag der Anmeldung:
01.12.82 Patentblatt 82/48

(45) Bekanntmachung des Hinweises auf die Patenterteilung:
09.01.85 Patentblatt 85/2

(45) Bekanntmachung des Hinweises auf die Entscheidung
über den Einspruch:
12.04.89 Patentblatt 89/15

(84) Benannte Vertragsstaaten:
CH DE FR GB IT LI

(73) Patentinhaber: HOECHST AKTIENGESELLSCHAFT,
Postfach 80 03 20, D-6230 Frankfurt am Main 80 (DE)

(72) Erfinder: Schubert, Hans, Dr., Hölderlinstrasse 56,
D-6233 Kelkheim (Taunus) (DE)
Erfinder: Baessler, Konrad, Dr., Drosselweg 1,
D-6230 Frankfurt am Main 80 (DE)

(56) Entgegenhaltungen:
EP-A- 0 000 542
DD-C- 81 113
DD-C- 95 573
DE-A- 2 634 419
DE-A- 2 639 700
FR-A- 2 319 610

Houben-Weyl "Methoden der Organischen Chemie", 4.
Auflage, VI/3, Seite 76,77
Helvetica Chimica Acta 4, 297, 1029 (1921) BIOS 1153,
Seite 17

(56) Entgegenhaltungen: (Fortsetzung)
Ullmann, 4. Auflage, Band 17, Seiten 409, 410 (1976)
Pharmazie 24 (1969), 419-421: Chem. Abstr. 71:123778h
Khim. Ind. (Sofia) 9 (1969), 398-400: Chem. Abstr.
73:45032k
Ind. Chem. Mfr. 9 (1971), 21, 22: Chem. Abstr. 75:63297w
J. Org. Chem. 45 (1980), 2263-2264

Die Akte enthält technische Angaben, die nach dem
Eingang der Anmeldung eingereicht wurden und die
nicht in dieser Patentschrift enthalten sind.

# Beschreibung

Aus der DE-OS 2 634 419 ist es bekannt, p-Nitrophenetol durch Umsetzung von p-Chlor-nitrobenzol mit Natronlauge in Ethanol in Gegenwart eines Phasentransferkatalysators herzustellen. Hierbei werden pro Mol p-Chlor-nitrobenzol 1,6 Mol Ethanol und 3 Mol 50%ige Natronlauge bei 90°C umgesetzt, wobei das Produkt in etwa 90%iger Ausbeute mit einem Schmelzpunkt von 53 bis 57°C anfällt. Als Nebenprodukt werden etwa 10% 4,4'-Dichlorazoxybenzol gebildet. Die Abtrennung dieses Azoxybenzols ist aufwendig, und auch die Reinheit des p-Nitrophenetols reicht für die meisten Anwendungsgebiete, insbesondere als Farbenvorprodukt, nicht aus.

Nach Houben-Weyl, Methoden der Organischen Chemie, VI/3, Seite 77 (1965), werden – unter Bezug auf die dortigen Referenzangaben Chem. Ber. 15, 1002 (1982) und Helv. 4, 297, 1029 (1921) – die Ausbeuten an Nitrophenoläthern häufig durch die reduzierende Wirkung der Alkalialkoholate, welche zur Bildung von Azoxyverbindungen Veranlassung gäben, ungünstig beeinflusst, und durch Verdünnung mit Wasser oder Einhaltung niedriger Temperaturen (unterhalb 70°C) sollen sich diese Nebenreaktionen weitgehend vermeiden lassen. Die in der Originalliteratur Helv. 4 durchgeführten Untersuchungen erfolgten in wässrigalkoholischer Lösung; aus den dortigen Ergebnissen ist ersichtlich, dass mit Wasser stärker verdünnte Reaktionslösungen zwar die Bildung von Azoxyverbindungen zurückdrängen, die Umsetzung und somit die Ausbeuten jedoch erheblich verringern (loc. cit. Seite 309; analog für das p-Methoxy-nitrobenzol Seite 1033 ff.).

Es wurde nun gefunden, dass p-Nitrophenetol durch Umsetzung von p-Chlor-nitrobenzol mit Ethanol und Alkalihydroxid in Gegenwart eines Phasentransferkatalysators in vorzüglicher Reinheit und hoher Ausbeute erhalten wird, wenn man die Umsetzung von p-Chlor-nitrobenzol und Ethanol in Gegenwart von festem Natrumhydroxid und unter Einsatz der Reaktanten p-Chlor-nitrobenzol, Ethanol und festes Natriumhydroxid im Molverhältnis von 1:(1,3–1,6):(1,3–1,5) umsetzt und die Umsetzung ohne weiteres Lösemittel bei einer Temperatur von 60 bis 70°C durchführt. Man erhält so in einer Ausbeute von etwa 95% ein Produkt vom Schmelzpunkt 58 bis 59°C, das praktisch frei von 4,4'-Dichlor-azoxybenzol, p-Chlor-nitrobenzol 4,4'-Diethoxy-azobenzol und p-Nitrophenol ist. Die Reaktionstemperatur liegt vorzugsweise im Bereich von 65 bis 70°C.

Obgleich das Natriumhydroxid in fester Form, beispielsweise in Form von Plätzchen, Schuppen, Pulver oder Prills, in die Reaktion eingesetzt wird, werden überraschenderweise weder Nebenprodukte, wie insbesondere 4,4'-Dichlorazoxybenzol, gebildet noch wird die Ausbeute oder Reinheit des erwünschten p-Nitrophenetol erniedrigt. Der Einsatz von festem Natriumhydroxid hat zusätzlich den Vorteil, dass die Menge an Ethanol als Gesamtlösemittel reduziert werden konnte, ohne dass befürchtet zu werden braucht, dass das eingesetzte p-Chlor-nitrobenzol nicht vollständig abreagiert und so Ausbeute und Reinheit erniedrigt werden.

Als Phasentransferkatalysator können die üblichen Produkte Verwendung finden, wie sie beispielsweise in der DE-OS 2 634 419 aufgeführt sind. Besonders bevorzugt ist Cocosalkyl-dimethyl-benzyl-ammoniumchlorid, das leicht aus seiner handelsüblichen etwa 50%igen wässrigen Lösung isoliert werden kann. Cocosalkyl steht für ein Gemisch von Alkylresten mit gerader Zahl von C-Atomen von $C_{12}$–$C_{18}$, wobei der $C_{12}$-Anteil etwa 50% beträgt.

In dem nachfolgenden Beispiel beziehen sich Teile und Prozentangaben auf das Gewicht, sofern nichts anderes vermerkt ist.

Beispiel

In einem Rührapparat wurde die Lösung von 67 Teilen Cocosalkyl-dimethyl-benzyl-ammoniumchlorid (das durch Abdestillieren von Wasser aus 125 g der handelsüblichen etwa 50%igen wässrigen Lösung erhalten wurde) in 322,5 Teilen 95%igem Ethanol vorgelegt, mit 787,8 Teilen p-Chlornitrobenzol versetzt und auf 70°C erhitzt. Anschliessend wurden unter kräftigem Rühren innerhalb von 4 Stunden 280 Teile Ätznatron (fest, in Form von Prills) gleichmässig eingetragen. Die Reaktionstemperatur darf während der gesamten Reaktionsdauer nicht über 70°C ansteigen. Nach beendeter Ätznatronzugabe wurde noch 4 bis 6 Stunden zur Vervollkommnung der Reaktion bei 70°C nachgerührt.

Der Ansatz wurde anschliessend wie folgt aufgearbeitet: Überschüssiges Ethanol wurde im Vakuum aus dem Reaktionsgemisch abdestilliert, der Destillationssumpf zweimal mit je 1000 Teilen Wasser bei 70°C ausgerührt, die Wasserphase abgetrennt, die geschmolzene, organische Phase in 1500 Teilen Wasser bis zum Erkalten gerührt, das p-Nitrophenetol abgesaugt und nacheinander mit 1 l Wasser, 1 l etwa 1%iger Salzsäure und 1 l Wasser gewaschen.

Molverhältnis von
p-Chlor-nitrobenzol : Ethanol : NaOH = 1 : 1,4 : 1,4

Ausbeute:
793 g = 95,0% d.Th., bez. auf p-Chlor-nitrobenzol
EP.: 57,8°C
Fp.: 58 bis 59,5°C
Gehalt an

| | |
|---|---|
| 4,4'-Dichlorazoxybenzol | <0,1% |
| p-Chlor-nitrobenzol | <0,1% |
| 4,4'-Diethoxy-azoxybenzol | <0,1% |
| p-Nitrophenol | ≦0,1% |

## Patentansprüche

1. Verfahren zur Herstellung von p-Nitrophenetol aus p-Chlor-nitrobenzol und Ethanol in Gegenwart von Alkalimetallhydroxid und Phasentransferkatalysator, dadurch gekennzeichnet, dass man p-Chlor-nitrobenzol und Ethanol in Gegenwart von festem Natriumhydroxid unter Einsatz der Reaktanten p-Chlor-nitrobenzol, Ethanol und festes Natriumhydroxid im Molverhältnis von 1:(1,3–1,6):(1,3–1,5) umsetzt und die Umsetzung ohne weiteres Lösemittel bei einer Temperatur von 60 bis 70°C durchführt.

2. Verfahren nach Anspruch 1, dadurch gekennzeichnet, dass man die Umsetzung bei einer Temperatur von 65 bis 70°C durchführt.

**Claims**

1. A process for the preparation of p-nitro-phenetole from p-chloro-nitrobenzene and ethanol in the presence of an alkali metal hydroxide and a phase transfer catalyst, characterized by that p-chloro-nitrobenzene and ethanol are reacted in the presence of solid sodium hydroxide with the use of the reactants p-chloro-nitrobenzene, ethanol and solid sodium hydroxide in the molar ratio of 1:(1.3–1.6):(1.3–1.5), and that the reaction is carried out without any other sol-

2. A process according to claim 1, characterized by that the raction is carried out at a temperature of from 65 to 70°C.

**Revendications**

1. Procédé pour préparer le p-nitro-phénétole à partir du p-chloro-nitro-benzène et de l'éthanol en présence d'un hydroxyde de métal alcalin et d'un catalyseur de transfert de phase, procédé caractérisé en ce qu'on fait réagir le p-chloro-nitro-benzène et l'éthanol en présence d'hydroxyde de sodium solide en utilisant les réactants, c'est-à-dire le p-chloro-nitro-benzène, l'éthanol et l'hydroxyde de sodium solide, dans un rapport molaire de 1:(1,3–1,6):(1,3–1,5) et on effectue la réaction sans autre solvant à une température de 60 à 70°C.

2. Procédé selon la revendication 1 caractérisé en ce qu'on effectue la réaction à une température de 65 à 70°C.